Europäisches Patentamt

European Patent Office

Office européen des brevets

(11) Publication number: **0 115 936**
**A1**

## EUROPEAN PATENT APPLICATION

(21) Application number: 84300403.7

(22) Date of filing: 24.01.84

(51) Int. Cl.³: **C 12 N 15/00**
**// C12R1/46, C12R1/19**

(30) Priority: 24.01.83 JP 9740/83
08.06.83 JP 103196/83
31.08.83 JP 159345/83
10.01.84 JP 2361/84

(43) Date of publication of application: **15.08.84**
**Bulletin 84/33**

(84) Designated Contracting States: **AT BE CH DE FR GB IT LI LU NL SE**

(71) Applicant: **KABUSHIKI KAISHA YAKULT HONSHA, 1-19, Higashishinbashi 1-chome, Minato-ku Tokyo 105 (JP)**

(72) Inventor: **Ishiwa, Hiromi, 1715-7, Josuihon-cho Kodaira, Tokyo (JP)**
Inventor: **Tsuchida, Nobuo, 1-18, Utsukushigaoka Midori-ku, Yokohama Kanagawa (JP)**
Inventor: **Mutai, Masahiko, 988, 4-chome Shimizu Higashiyamato, Tokyo (JP)**

(74) Representative: **Brewer, Leonard Stuart et al, Sanderson & Co. 97 High Street, Colchester Essex CO1 1TH (GB)**

(54) Chimeric plasmids.

(57) A chimeric plasmid comprising:
(a) a tetracycline resistant gene deriving from pAMα1, one of the plasmids retained by Streptococcus faecalis,
(b) an ampicillin resistant gene deriving from pACYCl77, one of the vectors applicable to Escherichia coli, and
(c) at least one of (i) an area at which replication originates deriving from said plasmid pAMα1, and (ii) an area at which replication originates deriving from said vector pACYCl77, said tetracycline resistant gene containing only one site at which said chimeric plasmid is recognized and cleaved by the restriction enzyme Ball and said ampicillin resistant gene containing only one site at which said chimeric plasmid is recognized and cleaved by the restriction enzymes BglI and PstI. The chimeric plasmid is useful as a vector for conveying a gene into a host cell, usually of a bacterium, and the host can then be employed in biosynthesis of industrial products.

CHIMERIC PLASMID

FIELD OF THE INVENTION

The present invention relates to a variety of chimeric plasmids which are useful as cloning vectors functioning at least for Escherichia coli and/or Bacillus subtilis and which can be synthesized from a plasmid retained by Streptococcus faecalis, one of the gram-positive bacteria, and a vector taken out of E. coli, one of the gram-negative bacteria. More specifically, the present invention relates to a chimeric plasmid which comprises (a) a tetracycline resistant gene (Tc) deriving from pAMα1, one of the plasmids retained by S. faecalis, (b) an ampicillin resistant gene (Amp) deriving from pACYC177, one of the vectors applicable to E. coli, and (c) either or both of the areas at which replication originates (hereinafter referred to as the origins):(OripAMα1) deriving from the aforementioned plasmid pAMα1 and (Ori177) deriving from the aforementioned plasmid pACYC177, the aforementioned tetracycline resistant gene containing only one site at which the chimeric plasmid is specifically cleaved by the restriction enzyme BalI and the aforementioned ampicillin resistant gene containing only one site at which the chimeric plasmid is specifically cleaved by the restriction enzymes BglI and PstI.

BACKGROUND OF THE INVENTION

Insofar as an in vitro gene manipulation technology is concerned, it is required that a vector which is accepted by a host is available to allow a foreign DNA to migrate in a cell of the host and to allow the foreign DNA to express the genetic information thereof in the cell of the host. The behaviour of a vector is clear, to a considerable extent, for the host-vector system in which Escherichia coli is employed, because efforts have been concentrated thereon so far. At present, however, much effort is being employed to develop host-vector systems which employ various microorganisms other than E. coli, such as Bacillus subtilis which is one of the microorganisms useful from the industrial view point, Actinomycetes which are microorganisms capable of producing antibiotics, and yeast which is widely employed for brewing.

The fundamental requirements for a vector are that the vector has a gene arrangement which is necessary for replication and that the vector has a recognition and cleavage site for a restriction enzyme at which a foreign DNA is inserted. From the practical viewpoint, however, various additional items tabulated below are required for a vector:

1.  Each of the specific restriction enzymes which can be employed for a specific gene manipulation has a recognition and cleavage site which is convenient in later steps in the specific gene manipulation process.

2.  The vector has a high efficiency in expression of genetic information.

3.  The vector has a marker gene which is necessary for detection of a transformant.

4.  The vector is readily accepted by a specific host, and the vector has a convenient host range.

5.  The vector remains stable in a specific host without incurring disturbances.

6.  The vector permits a biological confinement which is aimed at reducing the possibility of any potential biological hazards.

This is the reason why only a few successful cases have been reported for vectors even within the category of host-vector systems employing E. coli or B. subtilis.

In the light of the aforementioned considerations, we have been using our best efforts to prepare plasmid vectors useful for the ultimate purpose of developing host-vector systems which employ E. coli, the biological properties of which are well known, and B. subtilis, which is useful industrially to produce amylase etc. As a result, we have been successful in developing a variety of chimeric plasmids which are synthesized from a plasmid retained by Streptococcus faecalis, pAMαl, and a vector applicable to E. coli, pACYC177, and which are excellent vectors for gene manipulation employing E. coli or B. subtilis.

## SUMMARY OF THE INVENTION

The invention provides a chimeric plasmid comprising:

(a) a tetracycline resistant gene deriving from pAMαl, one

of the plasmids retained by Streptococcus faecalis,

(b) an ampicillin resistant gene deriving from pACYC177, one of the vectors applicable to Escherichia coli, and

(c) at least one of (i) an area at which replication originates deriving from said plasmid pAMα1, and

(ii) an area at which replication originates deriving from said vector pACYC177,

said tetracycline resistant gene containing only one site at which said chimeric plasmid is recognized and cleaved by the restriction enzyme BalI and said ampicillin resistant gene containing only one site at which said chimeric plasmid is recognized and cleaved by the restriction enzymes BglI and PstI.

The invention also provides a process for preparing the plasmid, which process comprises cleaving pAMα1, a plasmid retained by Streptococcus faecalis DS5 (ATCC14508), and pACYC177, a vector applicable to Escherichia coli, and ligating the cleaved DNA fragments of said plasmid and vector to include a tetracycline resistant gene (Tc) deriving from the aforementioned pAMα1, an ampicillin resistant gene (Amp) deriving from the aforementioned pACYC177 and either or both of: (i) an area at which replication originates deriving from said plasmid pAMα1, and (ii) an area at which replication originates deriving from said vector pACYC177.

The invention provides also a method employing a vector to convey a gene into a host cell, usually of a microorganism, especially a bacterium, which may be a gram-positive or gram-negative bacterium, especially <u>Escherichia</u> <u>coli</u> or <u>Bacillus</u> <u>subtilis</u>, in which method the present chimeric plasmid is used as vector.

The invention provides also of course the host produced by this method (including the descendents of the immediate host).   The invention also provides biosynthesis employing a gene cloned by this host.

The invention also provides a microorganism as deposited at the Fermentation Research Institute Agency of Industrial Science and Technology, Japan, under the deposition number FERM BP-436, FERM BP-437, FERM BP-438, FERM BP-439, FERM BP-441, FERM BP-442, FERM BP-443, FERM BP-444, FERM BP-445 or FERM BP-446.

The variety of chimeric plasmids in accordance with the present  invention is a variety of chimeric plasmids which can be produced by cleaving pAMα1, a plasmid retained by Streptococcus faecalis DS5 (ATCC14508), and pACYC177, a vector
applicable to Escherichia coli and by ligating the cleaved
DNA fragments of said plasmid and vector to include
a tetracycline resistant gene (Tc) deriving from the afore-
mentioned pAMα1, an ampicillin resistant gene (Amp) deriving
from the aforementioned pACYC177 and either or both of the
origin (OripAMα1) deriving from the aforementioned pAMα1 and
the origin (Ori177) deriving from the aforementioned pACYC177.

The chimeric plasmids included in the aforementioned
genus category are classified, following the difference in
the reduced molecular weight, into the 10 species
designated pHY780, pHY600, pHY460, pHY385, pHY360, pHY340,
pHY330, pHY310, pHY285 and pHY225.

Since each of the chimeric plasmids has
a tetracycline resistant gene and an ampicillin resistant
gene in its DNA, they have the capability of
providing a host with resistance against both tetracycline and
ampicillin during the transformation process applied to
E. coli. They also have the capability of
providing a host with resistance against tetracycline during
the transformation process applied to Bacillus subtilis.
This genetic nature can function as a marker for
detection and selection of a transformant or a host which
retains a chimeric plasmid in which a desired foreign DNA
is to be inserted. Further, since the tetracycline resistant gene of any of the chimeric plasmids in accordance with the present invention contains      only one site at which
the chimeric plasmid is recognized and cleaved by the
restriction enzyme BalI, and also since the ampicillin
resistant  gene of any of the same chimeric plasmids
contains     only one site at which the chimeric plasmid
is recognized and cleaved by either of the restriction
enzymes BglI and PstI, no more fragments than are required
are produced from any of the chimeric plasmids in accordance
with the present invention, provided the restriction enzymes
employed for the cleavage are limited to the aforementioned
ones.

Accordingly, a foreign DNA can be inserted in one of
the aforementioned single sites. at which the chimeric
plasmid is recognized and cleaved by one of the aforementioned restriction enzymes.  The tetracycline resistant
gene is cleaved by the restriction enzyme BalI to form a
pair of flush ends at the centre of the 6-nucleotide sequence
of the DNA.  Thus, it is possible, without utilizing T4 DNA
polymerase or S1 nuclease, to ligate the aforementioned
flush ends with any of the foreign DNA fragments each of
which has a pair of flush ends.  No limitations are imposed
on the method of production of the foreign DNA-s having
flush ends.  In other words, not only the aforementioned
restriction enzymes but also any other restriction enzymes
which produce flush ends can be employed for preparation of
the foreign DNA-s.  Further, it is possible to produce a
ligated DNA employing foreign DNA fragments having artificially produced flush ends.

Out of the chimeric plasmids in accordance with the present invention, pHY460, pHY385, pHY360, pHY340 and pHY310      function as shuttle vectors which are applicable both to E. coli and B. subtilis.  This is because the aforementioned two independent origins, namely OripAMα1, the origin deriving from the plasmid, pAMα1, and Oril77, the origin deriving from the vector, pACYC177, both of which are contained in any of these chimeric plasmids in accordance with the present invention, are correctly interpreted and allowed to enjoy stable replications not only in E. coli but also in B. subtilis, thus being able to express the tetracycline resistant  gene at least in each of E. coli and B. subtilis.

The DNA cloning system whose research and development has been      most extensively achieved is the vector system (EK system) employing E. coli, one of the gram-negative bacteria and its vectors.  In accordance with this system, genes deriving from the gram-negative bacteria and some specific genes deriving from      gram-positive bacteria can be expressed.

In contrast, many problems still remain unsolved for the system employing      gram-positive bacteria.  This is the reason why a considerable amount of attention is paid to this subject.  Particularly, though B. subtilis is a group of useful microorganisms which have a number of practical advantages, their biological properties are    entirely different from those of E. coli.  Thus, the study of the genetic information of B. subtilis and the development of a cloning system employing the same bacteria have become matters of great interest.

In these circumstances, the industry is eager to develop a host-vector system employing B. subtilis. Therefore, our success in development of a variety of shuttle vectors which are applicable both to E. coli, one of the typical bacteria belonging to gram-negative bacteria, and to B. subtilis, one of the typical bacteria belonging to gram-positive bacteria, is very significant, because it is recognized as a milestone to reach a DNA cloning system employing gram-positive bacteria.  Accordingly, it is clear that the chimeric plasmids in accordance with the present invention are useful from the industrial viewpoint..

In addition, since possibilities may exist for utilization of chimeric plasmids in accordance with the present invention for the purposes of gene analysis and molecular breeding of    other gram-positive bacteria, e.g. those belonging to Lactobacillus, Bifidobacterium and the like, chimeric plasmids in accordance with the present invention are promising from this viewpoint of industrial application.

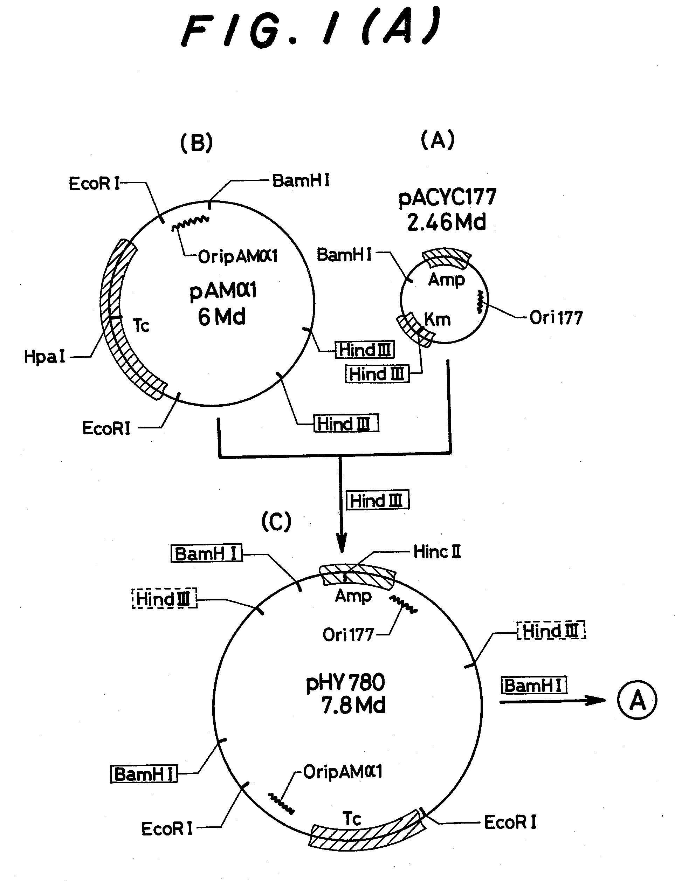

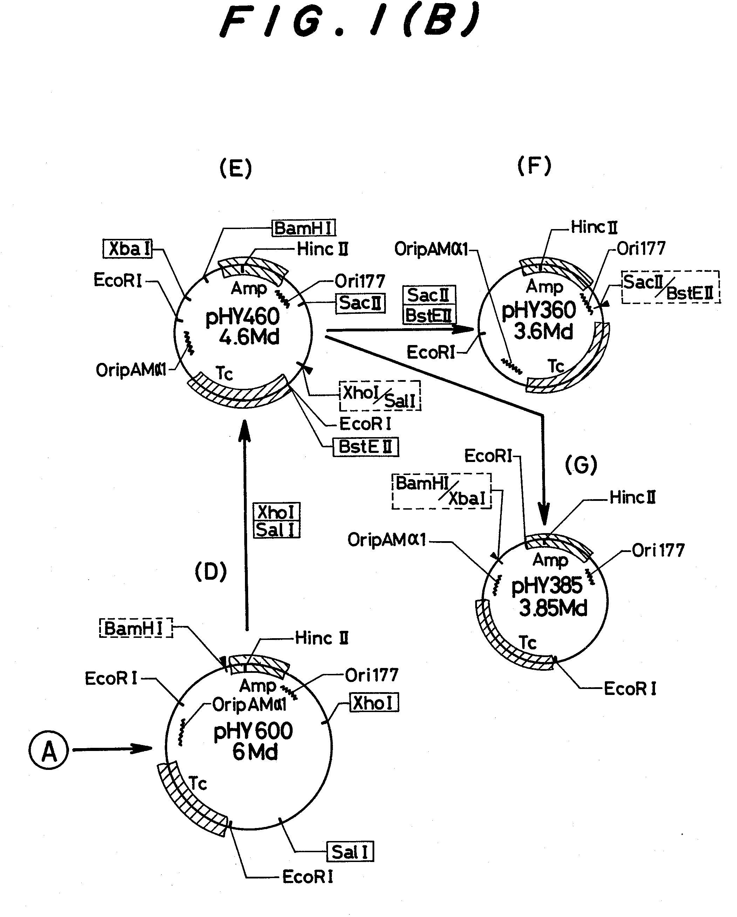

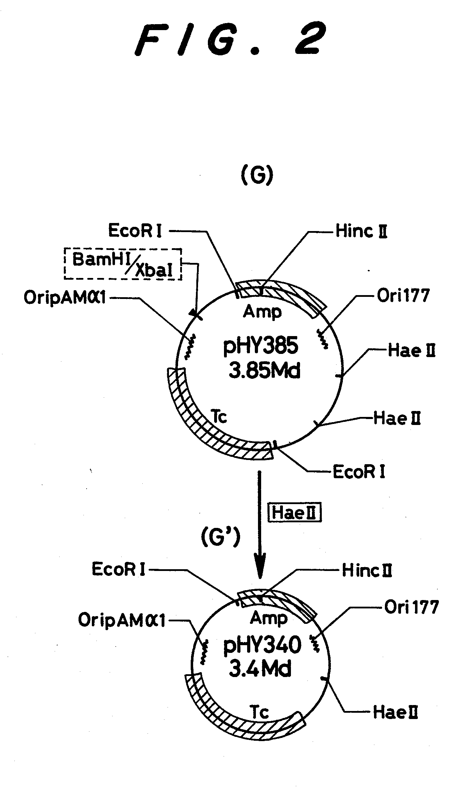

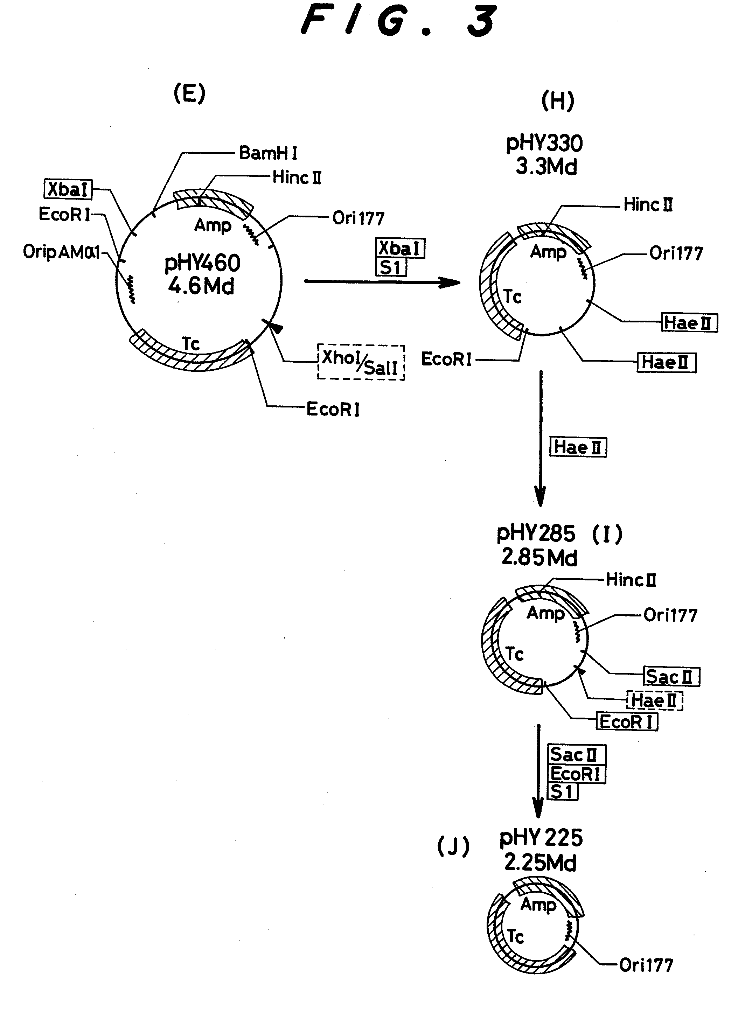

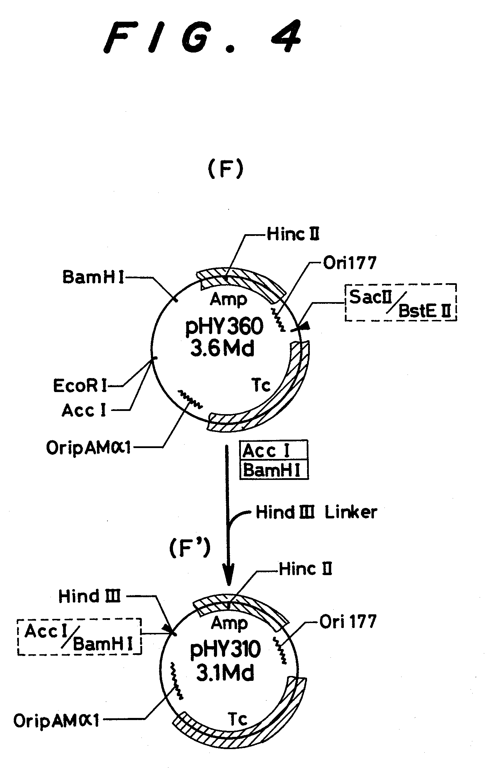

Figs. 1 to 3 show the synthesis progress of the specific chimeric plasmids in accordance with the present invention, which starts at the vector pACY177 (shown as (A) in Fig. 1(A)) and the plasmid pAMα1 (shown as (B) in Fig. 1(A)) to arrive at chimeric plasmids pHY385 (shown as (G) in Fig. 1(B)), pHY360 (shown as (F) in Fig. 1(B)), pHY340 (shown as (G') in Fig. 2), pHY330 (shown as (H) in Fig. 3), pHY310 (shown as (F') in Fig. 4), pHY285 (shown as (I) in Fig. 3) and pHY225 (shown as (J) in Fig. 3), passing through the chimeric plasmids pHY780 (shown as (C) in Fig. 1(A)), pHY600 (shown as (D) in Fig. 1(B)) and pHY460 (shown as (E) in Fig. 1(B)).

The character of each of the specific chimeric plasmids in accordance with the present invention will be described below:

Chimeric plasmid pHY780

(1)     The chimeric plasmid pHY780 is a circular deoxyribonucleic acid (DNA) whose      molecular weight is approximately 7.8 Md.

(2)     pHY780 is a chimeric plasmid which can be constructed by ligating a larger fragment of a linear DNA which is produced by digesting a plasmid pAMα1 containing a tetracycline resistant  gene (Tc) with a restriction enzyme HindIII and a fragment of a linear DNA which is produced by digesting a plasmid vector pACYC177 containing an ampicillin resistant  gene (Amp) and a kanamycin resistant  gene (Km) with the same enzyme HindIII.

The plasmid pAMα1 and the vector pACYC177 both of which are employed as      starting materials for the aforementioned synthesis process are publicly known
(pAMα1:  Proc. Natl. Acad. Sci. USA, 72, 1720-1724
(1975), pACYC177:  J. Bacteriol., 134, 1141-1156 (1978)).

(3) pHY780 includes an ampicillin resistant  gene (Amp)
which contains  only one site at which the chimeric
plasmid is recognized and cleaved by any of the
restriction enzymes BglI and PstI and a tetracycline
resistant  gene (Tc) which contains  only one site
at which the chimeric plasmid is recognized and cleaved
by the restriction enzyme BalI. Accordingly, these
recognition and cleavage sites can be utilized as the
insertional inactivation site at which a foreign DNA is
inserted.

The following table lists the number  of recognition and cleavage sites at which pHY780 is cleaved by
various restriction enzymes:

| Restriction Enzyme | Number of Recognition and Cleavage Sites |
|---|---|
| BalI | 1 |
| BamHI | 2 |
| BanI | 1 or more |
| BglI | 1 |
| EcoRI | 2 |
| EcoRV | 1 or more |
| HaeII | 4 |
| HindIII | 3 |
| HpaI | 2 |
| KpnI | 2 |

| Restriction Enzyme | Number of Recognition and Cleavage Sites |
|---|---|
| PstI | 1 |
| PvuI | 2 |
| SacII | 1 |
| SalI | 1 |
| XbaI | 1 |
| XhoI | 1 |

The number of recognition and cleavage sites recorded in the table were determined by counting the number of recognizable bands which were generated on an electrophoresis agarose gel by the DNA fragments produced by digestion of pHY780 with excess of various restriction enzymes.

(4) pHY780 replicates in E. coli, gram-negative bacteria. In addition, pHY780 gives a host the resistance deriving from the ampicillin resistant gene (Amp) and the tetracycline resistant gene (Tc), both of which exist in the specific regions on the DNA sequence of pHY780. This resistance can be utilized as a selection marker for a transformant.

Chimeric plasmid pHY600

(1) The chimeric plasmid pHY600 is a circular DNA whose molecular weight is approximately 6.0 Md.

(2) pHY600 is a chimeric plasmid which can be constructed by the following process:

(i) Restriction enzyme BamHI is employed to cleave the aforementioned pHY780 to shorten the DNA dimension thereof by depriving pHY780 of a DNA fragment whose molecular weight is approximately 1.8 Md.

(ii)    T4 ligase is employed to ligate    both cohesive ends of the shortened DNA fragment to produce a circular DNA.

(3)    As is in the case of pHY780, the pHY600 includes an ampicillin resistant  gene (Amp) which contains only one site at which the chimeric plasmid is recognized and cleaved by any of the restriction enzymes BglI and PstI and a tetracycline resistant gene (Tc) which contains    only one site at which the chimeric plasmid is recognized and cleaved by the restriction enzyme BalI.  Accordingly, these recognition and cleavage sites can be utilized as the insertional inactivation site at which a foreign DNA is inserted.

The following table lists the number   of recognition and cleavage sites at which pHY600 is cleaved by various restriction enzymes:

| Restriction Enzyme | Number of Recognition and Cleavage Sites |
|---|---|
| BalI | 1 |
| BamHI | 1 |
| BanI | 1 or more |
| BglI | 1 |
| EcoRI | 2 |
| EcoRV | 1 or more |
| HaeII | 2 |

| Restriction Enzyme | Number of Recognition and Cleavage Sites |
|---|---|
| HindIII | 1 |
| HpaI | 2 |
| PstI | 1 |
| PvuI | 2 |
| SacII | 1 |
| SalI | 1 |
| XbaI | 1 |
| XhoI | 1 |

(4)     As is in the case of pHY780, pHY600 replicates in E. coli, gram-negative bacteria. In addition, pHY600 gives a host the resistant deriving from the tetracycline resistant gene (Tc) and the ampicillin resistant gene (Amp). Accordingly, pHY600 can function as a cloning vector for at least E. coli.

Chimeric plasmid pHY460

(1)     The chimeric plasmid pHY460 is a circular DNA whose molecular weight is approximately 4.6 Md.

(2)     pHY460 is a chimeric plasmid which can be constructed by the following process:

(i)     Restriction enzymes XhoI and SalI are employed to cleave the aforementioned pHY600 to shorten the DNA dimension thereof by depriving pHY600 of a DNA fragment whose molecular weight is approximately 1.4 Md.

(ii)     T4 ligase is employed to ligate both cohesive ends of the shortened DNA fragment to produce a circular DNA.

(3)     pHY460 includes an ampicillin resistant gene (Amp) which contains only one site at which the chimeric plasmid is recognized and cleaved by any of the restriction enzymes BglI, PstI, BanI and PvuI and a tetracycline resistant gene (Tc) which contains only one site at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

The following table lists the number of recognition and cleavage sites at which pHY460 is cleaved by various restriction enzymes:

| Restriction Enzyme | Number of Recognition and Cleavage Sites |
|---|---|
| BalI | 1 |
| BamHI | 1 |
| BanI | 1 |
| BglI | 1 |
| BstEII | 1 |
| EcoRI | 2 |
| EcoRV | 1 |
| HaeII | 2 |
| HpaI | 1 |
| PstI | 1 |
| PvuI | 1 |
| SacII | 1 |
| XbaI | 1 |

(4)    pHY460 replicates not only in E. coli, gram-negative bacteria, but also in B. subtilis, gram-positive bacteria. In addition, pHY460 gives a host the resistance deriving from the tetracycline resistant gene. Accordingly, pHY460 can function as a cloning vector (a shuttle vector) at least both for E. coli and for B. subtilis.

Chimeric plasmid pHY360

(1)    The chimeric plasmid pHY360 is a circular DNA whose molecular weight is approximately 3.6 Md.

(2)    pHY360 is a chimeric plasmid which can be constructed by the following process:

(i)    Restriction enzymes SacII and BstEII are employed to cleave the aforementioned pHY460.

(ii)    The larger DNA fragment is digested with S1 nuclease to make both ends thereof flush.

(iii)    T4 ligase is employed to ligate both the flush ends of the DNA fragment to produce a circular DNA.

(3)    pHY360 includes an ampicillin resistant gene (Amp) which contains only one site at which the chimeric plasmid is recognized and cleaved by any of the restriction enzymes BglI, PstI, BanI and PvuI and a tetracycline resistant gene (Tc) which contains only one site at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

The following table lists the number of recognition and cleavage sites at which pHY360 is cleaved by various restriction enzymes:

| Restriction Enzyme | Number of Recognition and Cleavage Sites |
|---|---|
| BalI | 1 |
| BamHI | 1 |
| BanI | 1 |
| BglI | 1 |
| EcoRI | 1 |
| EcoRV | 1 |
| HpaI | 1 |
| PstI | 1 |
| PvuI | 1 |
| XbaI | 1 |

(4)   As is in the case of pHY460, pHY360 replicates not only in E. coli, gram-negative bacteria but also in B. subtilis, gram-positive bacteria. In addition, pHY360 gives a host the resistance against tetracycline. Accordingly, pHY360 can function as a cloning vector (a shuttle vector) at least both for E. coli and for B. subtilis.

A successive cultivation test was conducted for 50 generations to determine the stability of pHY360 in B. subtilis. The results of the test showed that approximately 10% of the transformants were resistant to tetracycline after the 50-generation successive cultivation test.

Chimeric plasmid pHY310

(1)     The chimeric plasmid pHY310 is a circular DNA whose molecular weight is approximately 3.1 Md.

(2)     pHY310 is a chimeric plasmid which can be constructed by the following process:

(i)     Restriction enzymes AccI and BamHI are employed to cleave the aforementioned pHY360.

(ii)    Both cohesive ends of the cleaved DNA fragment are converted into a pair of flush ends by treating them with T4 DNA polymerase and 4 kinds of deoxyribonucleoside triphosphate.

(iii)   T4 ligase is employed to ligate each of the flush ends of the larger DNA fragment (produced by digestion of pHY360) and each of the flush ends of a HindIII linker (dpCAAGCTTG), which bridges both the ends of the same DNA fragment to produce a circular DNA.

(3)     pHY310 includes an ampicillin resistant gene (Amp) which contains only one site at which the chimeric plasmid is recognized and cleaved by any of the restriction enzymes BglI, PstI, BanI and PvuI and a tetracycline resistant gene (Tc) which contains only one site at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

In addition, since on the sequence of the bridging HindIII linker, there exists only one site at which the chimeric plasmid is recognized and cleaved by the restriction enzyme HindIII, this recognition and cleavage site can also count as the position at which a foreign DNA is inserted.

The following table lists the number of recognition and cleavage sites at which pHY310 is cleaved by various restriction enzymes:

| Restriction Enzyme | Number of Recognition and Cleavage Sites |
|---|---|
| BalI | 1 |
| BanI | 1 |
| BglI | 1 |
| EcoRV | 1 |
| HincII | 3 |
| HindIII | 1 |
| HpaI | 1 |
| PstI | 1 |
| PvuI | 1 |

(4)     As is in the case of pHY360, pHY310 replicates not only in E. coli, gram-negative bacteria, but also in B. subtilis, gram-positive bacteria. In addition, pHY310 gives a host the resistance against tetracycline. Accordingly, pHY310 can function as a cloning vector (a shuttle vector) at least both for E. coli and for B. subtilis.

Chimeric plasmid pHY385

(1)  The chimeric plasmid pHY385 is a circular DNA whose molecular weight is approximately 3.85 Md.

(2)  pH385 is a chimeric plasmid which can be obtained by the following process:

(i)  A microorganism belonging to B. subtilis is transformed by the aforementioned pHY460.

(ii)  The transformants are cultivated for 50 successive generations to shorten the DNA dimension thereof by depriving pHY460 of a DNA fragment whose molecular weight is approximately 0.75 Md.

(3)  pHY385 includes an ampicillin resistant gene (Amp) which contains only one site at which the chimeric plasmid is recognized and cleaved by any of the restriction enzymes BglI, PstI, BanI and PvuI and a tetracycline resistant gene (Tc) which contains only one site at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

The following table lists the number of recognition and cleavage sites at which pHY385 is cleaved by various restriction enzymes:

| Restriction Enzyme | Number of Recognition and Cleavage Sites |
|:---:|:---:|
| BalI | 1 |
| BanI | 1 |
| BglI | 1 |
| BstEII | 1 |
| EcoRI | 2 |
| EcoRV | 1 |
| HaeII | 2 |
| HpaI | 1 |
| PstI | 1 |
| PvuI | 1 |
| SacII | 1 |

(4)    As is in the case of pHY460, pHY385 replicates not only in E. coli, gram-negative bacteria, but also in B. subtilis, gram-positive bacteria. In addition, pHY385 gives a host the resistance against tetracycline. Accordingly, pHY385 can function as a cloning vector (a shuttle vector) at least both for E. coli and for B. subtilis.

Chimeric plasmid pHY340

(1)    The chimeric plasmid pHY340 is a circular DNA whose molecular weight is approximately 3.4 Md.

(2)    pHY340 is a chimeric plasmid which can be constructed by the following process:

(i)    Restriction enzyme HaeII is employed to cleave the aforementioned pHY385 to shorten the DNA dimension thereof by depriving pHY385 of a DNA fragment whose molecular weight is approximately 0.45 Md.

(ii)    T4 ligase is employed to ligate    both cohesive ends of the shortened DNA fragment to produce a circular DNA.

(3)      pHY340 includes an ampicillin resistant  gene (Amp) which contains     only one site at which the chimeric plasmid is recognized and cleaved by any of the restriction enzymes BglI, PstI, BanI and PvuI and a tetracycline resistant  gene (Tc) which contains only one site at which the chimeric plasmid is recognized and cleaved by any of the restriction enzymes BalI, EcoRV and HpaI.  Accordingly, these recognition and cleavage sites can be utilized as the insertional inactivation site at which a foreign DNA is inserted.

The following table lists the number  of recognition and cleavage sites at which pHY340 is cleaved by various restriction enzymes:

| Restriction Enzyme | Number   of Recognition and Cleavage Sites |
|---|---|
| BalI | 1 |
| BanI | 1 |
| BglI | 1 |
| BstEII | 1 |
| EcoRI | 2 |
| EcoRV | 1 |
| HaeII | 1 |

| Restriction Enzyme | Number of Recognition and Cleavage Sites |
|---|---|
| HpaI | 1 |
| PstI | 1 |
| PvuI | 1 |
| SacII | 1 |

(4)     pHY340 replicates not only in E. coli, gram-negative bacteria, but also in B. subtilis, gram-positive bacteria.  In addition, pHY340 gives a host the resistance against tetracycline.  Accordingly, pHY340    can       function as a cloning vector (a shuttle vector) at least both for E. coli and for B. subtilis.

Chimeric plasmid pHY330

(1)     The chimeric plasmid pHY330 is a circular DNA whose       molecular weight is approximately 3.3 Md.

(2)     pHY330 is a chimeric plasmid which can be produced by the following process:

(i)     pHY460 is cleaved by the restriction enzyme XbaI to produce a linear DNA fragment, before the fragment is subjected to digestion with S1 nuclease to be shortened in random length and to have a pair of flush ends.

(ii)    T4 ligase is employed to ligate    both flush ends of the shortened DNA fragment to produce a circular DNA.

(3)     pHY330 includes an ampicillin resistant  gene (Amp) which contains     only one site at which the chimeric plasmid is recognized and cleaved by any of the restriction enzymes BglI, PstI, BanI and PvuI and a tetracycline resistant gene (Tc) which contains only one site at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

The following table lists the number of recognition and cleavage sites at which pHY330 is cleaved by various restriction enzymes:

| Restriction Enzyme | Number of Recognition and Cleavage Sites |
|---|---|
| BalI | 1 |
| BanI | 1 |
| BglI | 1 |
| BstEII | 1 |
| EcoRI | 1 |
| EcoRV | 1 |
| HaeII | 2 |
| HpaI | 1 |
| PstI | 1 |
| PvuI | 1 |
| SacII | 1 |

(4)     As is in the case of pHY760, pHY330 replicates in E. coli, gram-negative bacteria. In addition, pHY330 gives a host the resistance against tetracycline and ampicillin.  Accordingly, pHY330    can    function
as a cloning vector at least for E. coli.

This chimeric plasmid pHY330, as well as the
chimeric plasmids pHY285 and pHY225 which will be
described later, is devoid of the origin (OripAM∝1)
which derives from the plasmid pAM∝1 but retains the
origin (Ori177) which derives from the vector pACYC177.
Thus, pHY330 does not replicate in gram-positive
bacteria.

## Chimeric plasmid pHY285

(1)     The chimeric plasmid pHY285 is a circular DNA
whose     molecular weight is approximately 2.85 Md.

(2)     pHY285 is a chimeric plasmid which can be
constructed by the following process:

(i)     pHY330 is cleaved by the restriction enzyme HaeII
to shorten the DNA dimension thereof by depriving
pHY285 of a DNA fragment whose molecular weight is
approximately 0.45 Md.

(ii)    T4 ligase is employed to ligate     both cohesive
ends of the shortened DNA fragment to produce a
circular DNA.

(3)     pHY285 includes an ampicillin resistant  gene (Amp)
which contains     only one site at which the chimeric
plasmid is recognized and cleaved by any of the restriction enzymes BglI, PstI, BanI and PvuI and a tetracycline
resistant  gene (Tc) which contains     only one site
at which the chimeric plasmid is recognized and cleaved by any of the restriction enzymes BalI, EcoRV and HpaI. Accordingly, these recognition and cleavage sites can be utilized as the insertional inactivation site at which a foreign DNA is inserted.

The following table lists the number of recognition and cleavage sites at which pHY285 is cleaved by various restriction enzymes:

| Restriction Enzymes | Number of Recognition and Cleavage Sites |
|---|---|
| AccI | 1 |
| BalI | 1 |
| BanI | 1 |
| BglI | 1 |
| BstEII | 1 |
| EcoRI | 1 |
| EcoRII | 4 |
| EcoRV | 1 |
| HaeII | 1 |
| HpaI | 1 |
| PstI | 1 |
| PvuI | 1 |
| SacII | 1 |

(4) As is in the case of pHY330, pHY285 replicates in E. coli, gram-negative bacteria. In addition, pHY285 gives a host the resistance against tetracycline and ampicillin. Accordingly, pHY285 can function as a cloning vector at least for E. coli.

Chimeric plasmid pHY225

(1)     The chimeric plasmid pHY225 is a circular DNA whose    molecular weight is approximately 2.25 Md.

(2)     pHY225 is a chimeric plasmid which can be constructed by the following process:

(i)     Restriction enzymes SacII and EcoRI are employed to cleave the aforementioned pHY285 to shorten the DNA dimension thereof by depriving pHY285 of a DNA fragment whose molecular weight is approximately 0.6 Md.

(ii)    The shortened DNA fragment is digested with S1 nuclease to make    both ends thereof flush.

(iii)   T4 ligase is employed to ligate both the flush ends of the DNA fragment to produce a circular DNA.

(3)     pHY225 includes an ampicillin resistant  gene (Amp) which contains    only one site at which the chimeric plasmid is recognized and cleaved by any of the restriction enzymes BglI, PstI, BanI and PvuI; and a tetracycline resistant  gene (Tc) which contains  only one site at which the chimeric plasmid is recognized and cleaved by any of the restriction enzymes BalI, EcoRV and HpaI.  Accordingly, these recognition and cleavage sites can be utilized as the insertional inactivation site at which a foreign DNA is inserted.

The following table lists the number  of recognition and cleavage sites at which pHY225 is cleaved by various restriction enzymes:

| Restriction Enzymes | Number of Recognition and Cleavage Sites |
|---|---|
| BalI | 1 |
| BanI | 1 |
| BglI | 1 |
| BstEII | 1 |
| EcoRII | 2 |
| EcoRV | 1 |
| HincII | 3 |
| HpaI | 1 |
| PstI | 1 |
| PvuI | 1 |

(4)     As is in the case of pHY285, pHY225 replicates in E. coli, gram-negative bacteria. In addition, pHY225 gives a host the resistance against tetracycline and ampicillin. Accordingly, pHY225     can     function as a cloning vector at least for E. coli.

Each of the aforementioned chimeric plasmids in accordance with the present invention, namely pHY780, pHY600, pHY460, pHY385, pHY360, pHY340, pHY330, pHY310, pHY285 and pHY225, fully satisfies the requirements for a vector useful for cloning an arbitrary gene at least in the DNA recombination technology employing E. coli as a host. In other words, they have the function to convey an arbitrary gene picked up out of other microorganisms and the like, into a host to retain and express the same gene therein. As a result, each of the chimeric plasmids in accordance with the present invention can be utilized as a vector for a type of DNA recombination technology, wherein a vector is caused to pick up the specific gene which has the capacity to be involved with bio-synthesis of a useful material or with the adjustment of bio-synthesis of a useful material out of a microorganism or the like, to convey the gene toward a host including at least E. coli, and to retain the gene in the microorganism, whereby the host can clone the specific gene to express its genetic information therein. Further, each of the chimeric plasmids in accordance with the present invention can be utilized for strengthening the bio-synthesis system by means of the function to amplify the genetic information specified by a specific gene, thereby enabling the productivity to be improved for the aforementioned useful material. Particularly, pHY460, pHY385, pHY360, pHY340 and pHY310 fully satisfy the requirements for a shuttle vector which is applicable at least both to E. coli and B. subtilis during the process of the aforementioned DNA recombination technology. In other words, these plasmids have, in addition to the function described above, the function to convey a gene from one host back to another host. As a result, each of these chimeric plasmids can be utilized as a shuttle vector for a type of DNA recombination technology in a much more convenient manner than was described above.

Further, pHY460, pHY385, pHY360, pHY340 and pHY310 can be utilized for expressing various genes deriving from some other microorganisms or the like, in gram-positive bacteria of which a typical example is B. subtilis. Therefore, these chimeric plasmids can provide means effective for gene analysis and molecular breeding of gram-positive bacteria.

BRIEF DESCRIPTION OF THE DRAWINGS

The present invention, together with its various features and advantages, can be readily understood from the following more detailed description presented in conjunction with the following drawings, in which:

Fig. 1(A) is a process chart showing the synthesis of one of the chimeric plasmids in accordance with the present invention, pHY780,

Fig. 1(B) is a process chart showing the synthesis of some of the chimeric plasmids in accordance with the present invention, pHY600, pHY460, pHY360 and pHY385,

Fig. 2 is a process chart showing the synthesis of one of the chimeric plasmids in accordance with the present invention, pHY340,

Fig. 3 is a process chart showing the synthesis of some of the chimeric plasmids in accordance with the present invention, pHY330, pHY285 and pHY225,

Fig. 4 is a process chart showing the synthesis of one of the chimeric plasmids in accordance with the present invention, pHY310, and

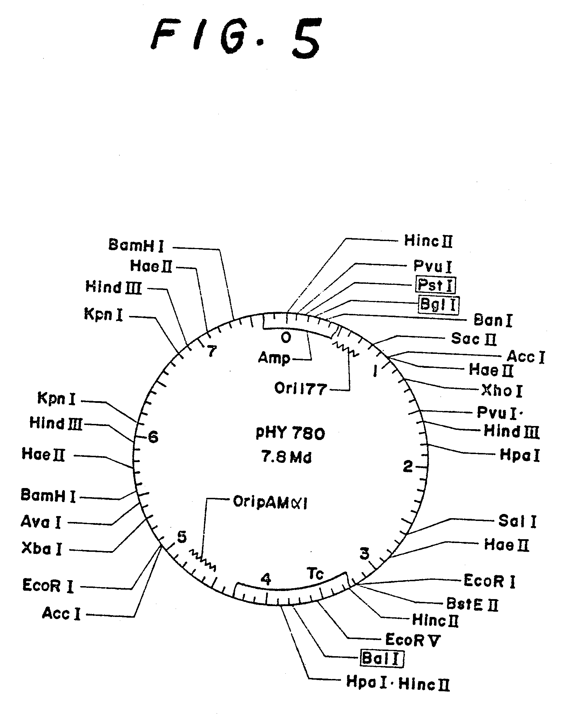

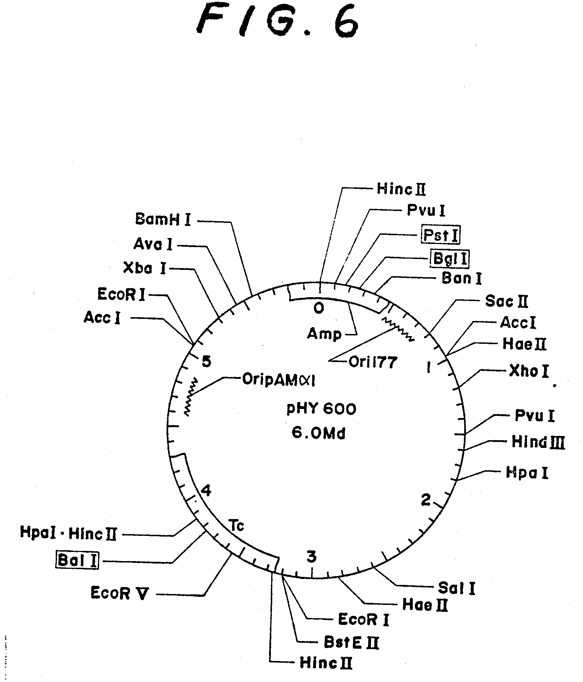

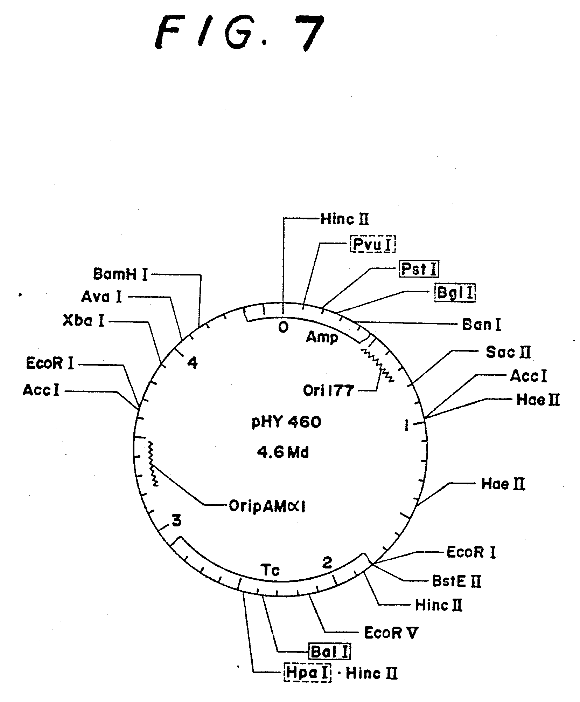

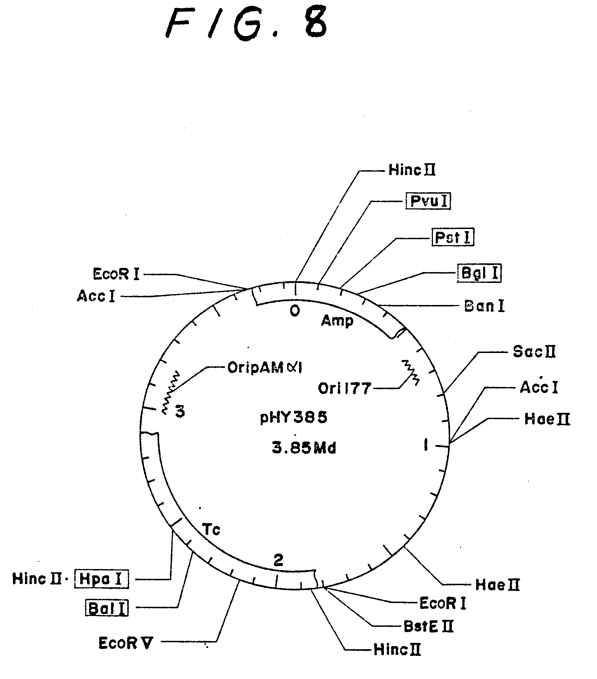

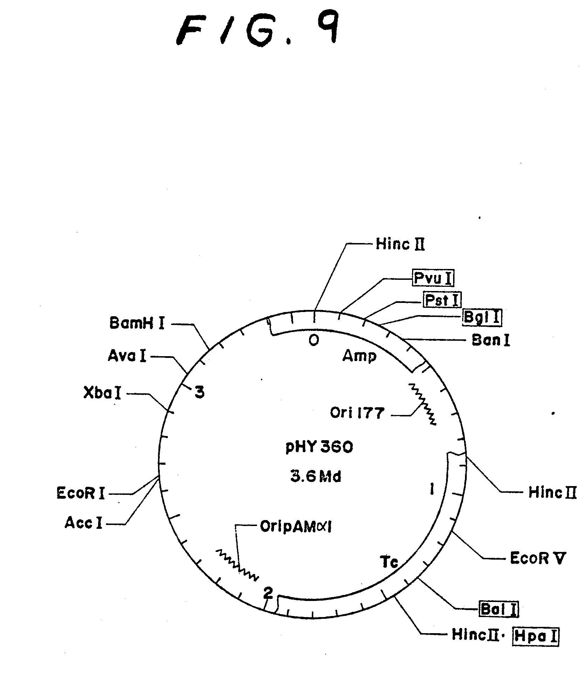

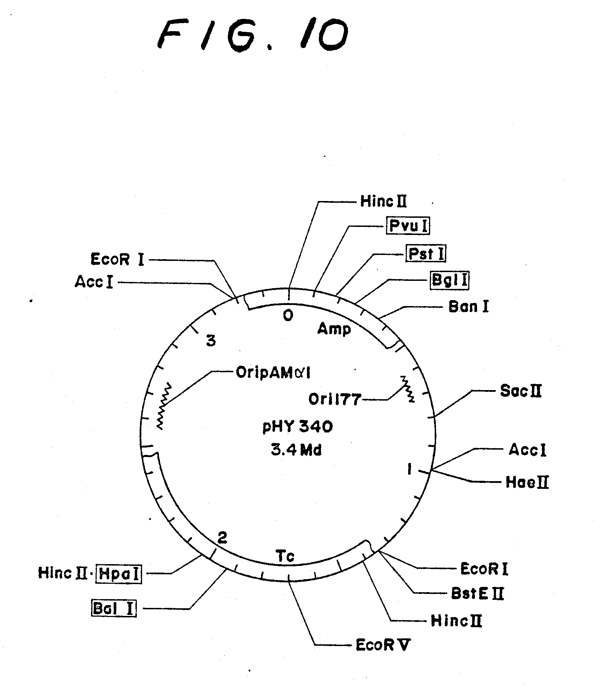

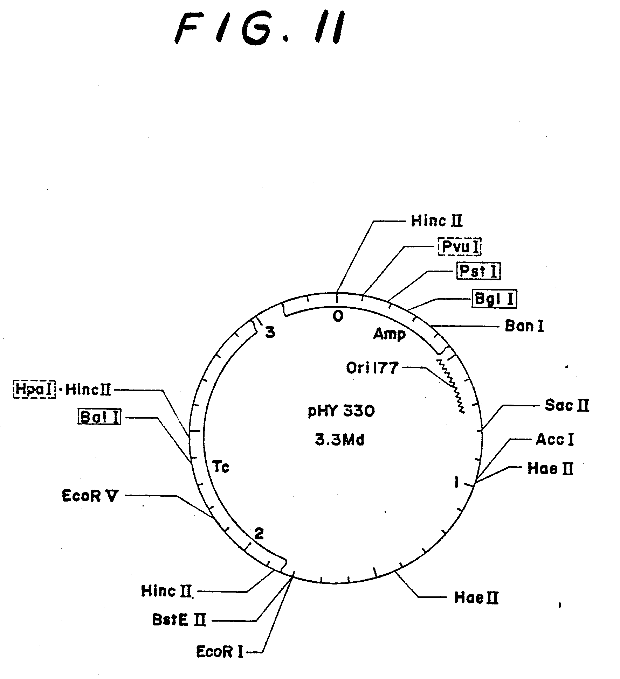

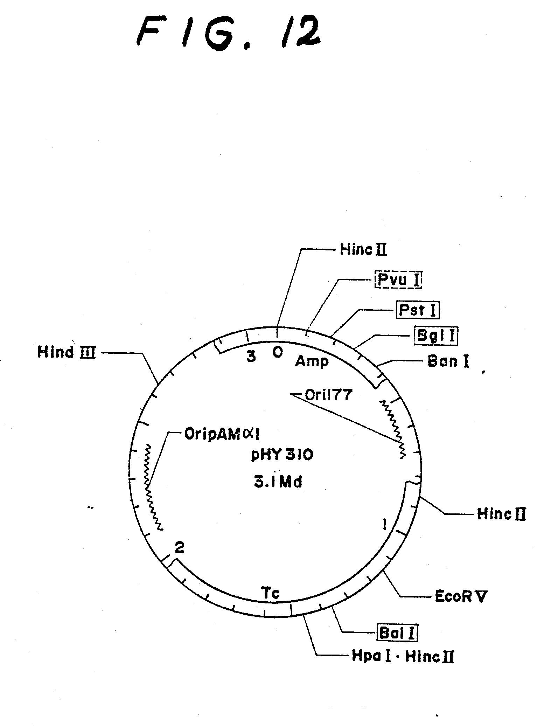

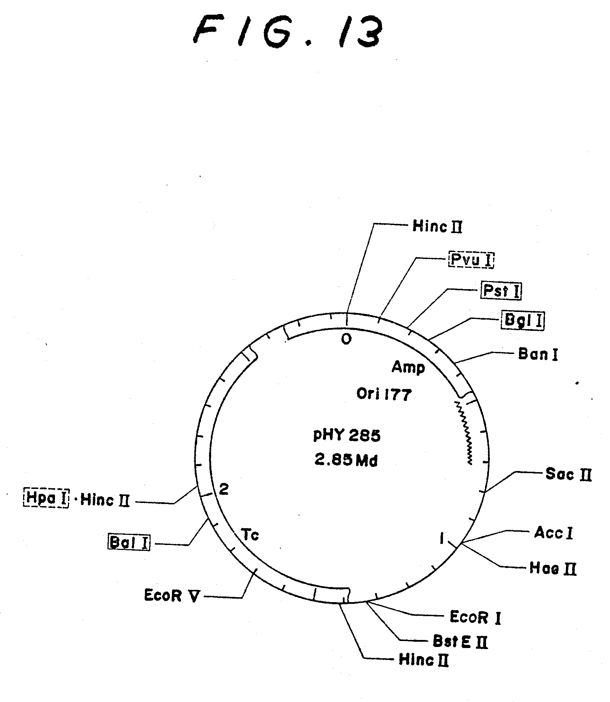

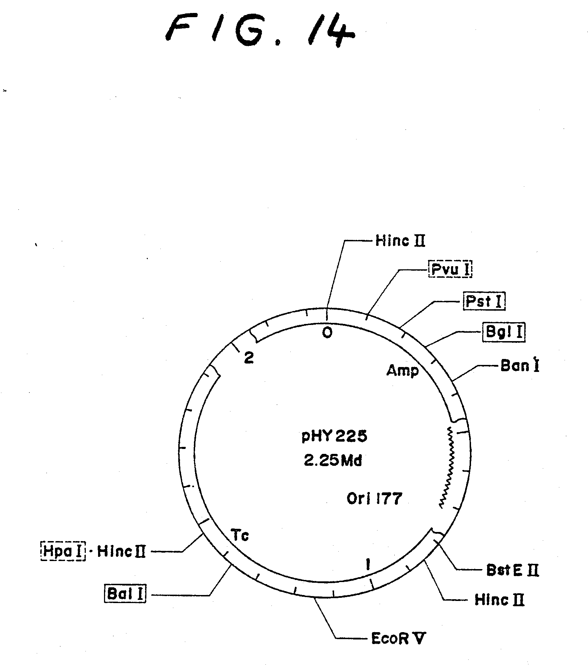

Figs. 5-14 show cleavage maps respectively of pHY 780, pHY 600, pHY 460, pHY 385, pHY 360, pHY 340, pHY 330, pHY 310, pHY 285 and pHY 225.

## DETAILED DESCRIPTION

A more detailed description will now be presented for specific chimeric plasmids in accordance with embodiments of the present invention.

EMBODIMENT 1    CHIMERIC PLASMID, pHY780

(1)    Preparation of pACYC177

E. coli K12 WA802r⁻m⁻ (pACYC177) (owned by Dr. H. Saito, Research Institute for Applied Microorganisms, Tokyo University) was cultivated overnight in L-broth (A broth produced by adjusting the pH value of the mixture containing 1% of bactotrypton, 0.5% of yeast extract, 0.5% of NaCl, 0.1% of glucose, to 7.0 employing 1N-NaOH). The cultivated bacteria were harvested by centrifugation before they were twice washed with 100 ml of 20 mM Tris - 10 mM EDTA (pH 8.0). The washed bacteria were resuspended in 9 ml of 20 mM Tris - 10 mM EDTA. Lysozyme and RNase were added to the suspension in the quantity required to make respectively 100 μg/ml and 50 μg/ml, before lysing reaction is allowed to occur at 0°C for 10 minutes. The bacteria which were subjected to the aforementioned lysing reaction were admixed with 1 ml of 2% SDS and were further lysed at 37°C for 5 minutes. After being kept at 0°C for 10 minutes, the solution was centrifuged at 36,000 rpm for 30 minutes to collect the supernatant thereof.

Cesium    chloride-ethidium bromide density gradient centrifugation was applied to the supernatant or a DNA crude sample of the desired DNA, in order to obtain the desired DNA out of the DNA crude sample.

For this purpose, the DNA crude sample was admixed with the crystalline cesium chloride in the quantity identical to that of the DNA crude sample and with 0.7 ml of ethidium bromide having a concentration of 5 mg/ml to prepare a solution containing the desired cccDNA, pACYC177. The solution was centrifuged twice at 36,000 rpm for 40 hours to collect the cesium chloride fractions containing the desired cccDNA.

The collected cesium chloride fractions containing the desired cccDNA were washed with isopropanol saturated with cesium chloride to exclude ethidium bromide. The cccDNA dissolved in the cesium chloride solution was dialysed against a buffer A containing 10 mM tris - 0.1 mM EDTA and having the pH value 7.4, before the dialysed cccDNA dissolved in the buffer A was admixed with phenol saturated with the same buffer, in the quantity required to increase the entire volume twice. The mixture was shaken to allow a small quantity of protein to be denatured, before the protein was allowed to travel to the boundary portion sandwiched between two liquid layers, the upper layer containing the desired cccDNA and the lower layer consisting of the phenol, which were separated from each other by centrifugation in order to collect the desired cccDNA. The upper layer was further dialysed against the buffer A to exclude the phenol which contaminated the desired cccDNA, to purify the desired cccDNA, pACYC177.

By the foregoing processes, pACYC177 whose cleavage map is shown by (A) in Fig. 1(A) was prepared.

(2)  Preparation of pAMα1

S. faecalis DS5 (ATCC14508) was cultivated overnight in Rogosa's    medium (a medium having the pH value    6.8 and of which 1 litre contains 20 g of glucose, 10 g of trypticase peptone, 5 g of yeast extract, 3 g of tryptose, 3 g of $K_2HPO_4$, 3 g of $KH_2PO_4$, 2 g of ammonium citrate, 1 g of sodium acetate, 1 g of Tween 80, 0.575 g of $MgSO_4 \cdot H_2O$, 0.5 g of L-cystine hydrochloride, 0.12 g of $MnSO_4 \cdot 2H_2O$ and 84 mg of $FeSO_4 \cdot 7H_2O$).

Processes similar to those which have been described above in (1) of this section for preparation of pACYC177, were employed to collect the DNA crude sample of DNA-s retained by S. faecalis.

The DNA sample which had been obtained from the DNA crude sample employing cesium chloride-ethidium bromide density gradient centrifugation, was further applied to sucrose density gradient centrifugation in order to selectively obtain the desired cccDNA, pAMα1 from the mixture containing pAMβ1 and pAMγ1 other than pAMα1.

Namely, 0.5 ml of the DNA sample was put on 12 ml of sucrose solution having the 5 - 30% density gradient which was prepared in a nitrocellulose tube.  The buffer which was employed in this process  was a mixture of 50 mM Tris - 50 mM EDTA - 50 mM NaCl, (pH 8.0).  Centrifugation was employed at 10°C  at 20,000 rpm for 16 hours to split the DNA sample to a plurality of DNA groups each of which contains a part of the DNA sample having a specific molecular weight. To collect each of the DNA groups individually, an opening was produced at the bottom of the nitrocellulose tube and a plurality of fractions consisting of 10 drops were collected. 0.8%- agarose gel electrophoresis process was applied to 10 $\mu$l each of the aforementioned fractions in order    to determine the molecular weight of the DNA-s contained in the specific fraction. The fractions containing the DNA-s having the molecular weight    6Md were selectively collected, before they were dialysed against the buffer A.

In this manner, pAM$\alpha$1 whose cleavage map is  shown by (B) in Fig. 1(A), was prepared.

(3) Cleavage of pACYC177 and pAM$\alpha$1 and Ligation of the cleaved DNA fragments of pACYC177 and pAM$\alpha$1

1 $\mu$l of a buffer (a mixture containing 100 mM of Tris - HCl having the pH value    7.6, 70 mM of $MgCl_2$, 70 mM of $\beta$ - mercaptoethanol and 500 mM of NaCl) and 1 $\mu$l of HindIII having an  activity of 4 units/$\mu$l were dropped in 10 $\mu$l each of the DNA solution (containing 0.1 - 0.2 $\mu$g of the DNA-s) produced and collected employing the foregoing processes described in (1) and (2), to allow HindIII to digest the DNA-s at 37°C. As a result,    both DNA-s contained in the aforementioned DNA solution (pACYC177 and pAM$\alpha$1) were recognized and cleaved at the recognition and cleavage site of HindIII. After digestion for 60 minutes,

the mixture was heated at 60°C for 10 minutes to terminate the digestion. After the mixture was admixed with 5 M of NaCl by the 1/50-volume and further with ethanol at the temperature of -20°C in the quantity twice as much as the entire quantity of the mixture, it was kept at -20°C for 30 minutes. After the mixture was centrifuged at 0°C at a speed of 15,000 rpm for 5 minutes, the precipitate was collected, before it was washed with ethanol of -20°C. After the washed precipitate was centrifuged at 0°C at 15,000 rpm for 2 minutes, it was entirely dried.

The DNA fragments acquired by the foregoing process were dissolved in 20 $\mu$l of sterilized water. The water containing the DNA fragments was admixed with a mixture containing 3 $\mu$l of 10 mM ATP, 3 $\mu$l of 100 mM dithiothreitol, 3 $\mu$l of 660 mM tris - HCl (pH 7.6) - 66 mM $MgCl_2$ and 0.5 $\mu$l of T4 ligase having an activity of 400 units/$\mu$l. Ligated in this process were a linear DNA fragment which was produced by allowing HindIII to digest pACYC177 and each of two linear DNA fragments, a smaller one and a larger one, which were produced by allowing the same restriction enzyme to digest pAM$\alpha$1. After this ligation process was allowed to occur at 15°C overnight, 120 $\mu$l of sterilized water was poured into the mixture until the entire quantity was increased to 150 $\mu$l.

(4)  Transformation of Escherichia coli

E. coli K12 C600r⁻m⁻ (owned by Dr. H. Saito, Research Institute for Applied Microorganisms, Tokyo University) was cultivated overnight in L-broth. 0.05 ml of this culture was inoculated in 5 ml of L-broth, to cultivate the same microorganism on a shaker at 37°C for 130 minutes. The culture
was centrifuged to collect the cells. After the cells were
washed with 0.1 M of $CaCl_2$ solution at 0°C, the washed cells
were suspended in 0.25 ml of the 0.1 M $CaCl_2$ solution at 0°C
to produce competent cells of the aforementioned microorganism.
0.1 ml each of the competent cells and the ligated DNA-s
produced employing the foregoing process described in (3),
were put together with each other and kept at 0°C for 5 minutes
to allow the competent cells to be transformed with the aforementioned ligated DNA-s. The transformants were cultivated
at 37°C for one hour in 1-ml of a culture containing 0.8 ml
of L-broth. The culture was applied to a medium containing
L-broth as well as agar, tetracycline and ampicillin in the
respective quantities of 1.5% of the L-broth volume, 20 $\mu$g/ml
and 30 units/ml, to be cultivated overnight at 37°C in
order to see that the transformants had actually obtained
the resistance against tetracycline and/or ampicillin. Since
the transformants were successfully cultivated on the medium
containing tetracycline and ampicillin, it was determined
that the transformants had readily obtained the resistance
against tetracycline and ampicillin. 10 colonies which grew
on the medium containing tetracycline and ampicillin were
picked up, and the picked up transformants were subjected to
a series of examinations described below to determine the
dimension of the plasmid DNA retained therein.

(5)  Extraction of the retained plasmid DNA and measurement of the molecular weight thereof

The aforementioned transformants were cultivated overnight in L-broth. Centrifugation was applied to 5 ml of the culture to selectively collect the transformants. The separated transformants were suspended in 0.5 ml of 50 mM tris - 10 mM EPTA (pH 7.4). This suspension was admixed with 0.2 ml of a mixture containing 2 mg/ml of lysozyme and 0.5 mg/ml of RNase to lyse the transformants at 37°C for 5 minutes. The solution was further admixed with 0.2 ml of 2% SDS solution to further lyse the transformants completely at 30°C for 1 to 2 minutes. After being kept at 0°C for 10 minutes, this solution was centrifuged at 0°C at 20,000 rpm for 10 minutes. 0.5 ml of the supernatant was carefully mixed with 0.5 ml of phenol saturated with buffer A to allow the undesired protein to be denatured with the phenol. The mixture was centrifuged at room temperature at 15,000 rpm for 3 minutes, and the upper layer which was separated from the lower layer was collected. 50 $\mu$l of the collected upper layer was applied with electrophoresis process on a 0.8%-agarose gel to determine the molecular weight of the plasmid DNA retained in each transformant.

The aforementioned sequencial process for determination of the molecular weight of a material was applied to 10 independent colonies of the transformants. The results showed the molecular weight of the plasmid DNA was approximately 7.8 Md.

In this manner, a new chimeric plasmid whose cleavage map is shown by (C) in Fig. 1(A) was synthesized from pACYC177 whose cleavage map is shown by (A) in Fig. 1(A) and pAMα1 whose cleavage map is shown by (B) in Fig. 1(A). This new chimeric plasmid was named pHY780. Another chimeric plasmid which was simultaneously synthesized in the same process was named pHY11.

The foregoing description confirms that pHY780 exhibits       stable replications and expression of genetic information thereof at least in E. coli.

Ligation of the DNA fragments of pACYC177 and of pAMα1 produces two independent chimeric plasmids, pHY780 and pHY11, whose molecular weights are the same as each other, due to alternative directions in which the DNA fragments are ligated. However, a cleaving process by means of BamHI allows the two independent chimeric plasmids to be sorted from each other, because the BamHI cleaving process produces two independent groups each of which is of dimension different from each other.

Further investigation was carried out for a variety of restriction enzymes which can be employed for removal of a portion or portions of each of pHY780 and pHY11 without losing at least both of the origins, OripAMα1 and Ori177, and also without causing any damage to two independent genes, (Tc) and (Amp). A larger variety of restriction enzymes available for pHY780, made it reasonable to choose pHy780, because this allowed easier shortening processes to follow.

EMBODIMENT 2     CHIMERIC PLASMID, pHY600

(1) Cleavage of pHY780 and ligation of the cleaved DNA fragment of pHY780

A transformed E. coli C600r⁻m⁻ (pHY780) produced in EMBODIMENT 1, was subjected to an extraction process similar to that which has been described in (5) for EMBODIMENT 1, in order to extract the retained plasmid DNA.

2 $\mu$l of a buffer (a mixture containing 100 mM of Tris - HCl having the pH value 7.6, 70 mM of $MgCl_2$, 70 mM of $\beta$-mercaptoethanol and 500 mM of NaCl) and BamHI of 4 units were dropped in 1 $\mu$g/20 $\mu$l of the extracted DNA, and BamHI was allowed to digest the extracted DNA at 37°C. After the digestion had lasted for 60 minutes, the digested DNA was heated at 60°C for 10 minutes to terminate the digestion. After the digested DNA was admixed with 80 $\mu$l of water, 2 $\mu$l of 5 M NaCl and 200 $\mu$l of ethanol at the temperature of -20°C, it was kept at -20°C for 30 minutes. The digested DNA was centrifuged at 0°C at 10,000 rpm for 5 minutes to collect a desired DNA. After the collected DNA was washed with 200 $\mu$l of ethanol at -20°C, the DNA was dried. The dried DNA was dissolved in 20 $\mu$l of water, and was ligated employing T4 ligase in the similar manner as described in (3) for EMBODIMENT 1.

(2) Transformation of Escherichia coli

E. coli C600r⁻m⁻ was transformed with the aforementioned plasmid DNA. The transformants showed the resistance against tetracycline having a concentration of 20 $\mu$g/ml and against ampicillin having a concentration of 30 units/ml.

A sequencial process for determination of the molecular weight of the DNA-s retained in the transformants, which was similar to that which has been described in (5) for EMBODIMENT 1, was applied to 10 independent colonies of the aforementioned transformants. The results showed the molecular weight of any of the plasmids was approximately 6 Md.

In this manner, a new chimeric plasmid whose cleavage map is shown by (D) in Fig. 1(B) was synthesized from pHY780 whose cleavage map is shown by (C) in Fig. 1(A). This new chimeric plasmid was named pHY600.

The results of the foregoing processes confirm that pHY600 can function as a cloning vector at least for E. coli.

EMBODIMENT 3    CHIMERIC PLASMID, pHY460

(1) Cleavage of pHY600 and ligation of the cleaved fragment of pHY600

A process similar to that which has been described in (3) for EMBODIMENT 1 was employed to allow the restriction enzymes XhoI and SalI to cleave pHY600, the product of EMBODIMENT 2, and to allow T4 ligase to ligate the cleaved DNA fragment thereof. A process similar to that which has been described in (4) for EMBODIMENT 1 was employed to transform E. coli with the ligated DNA and to produce a chimeric plasmid, which has a molecular weight of approximately 4.6 Md.

In this manner, a new chimeric plasmid whose cleavage map is shown by (E) in Fig. 1(B) was synthesized from pHY600 whose cleavage map is shown by (D) in Fig. 1(B). This new chimeric plasmid was named pHY460.

(2) Transformation of <u>Bacillus subtilis</u>

  (i) <u>Preparation of competent cells</u>

      <u>B. subtilis</u> Marburg 168 (owned by Dr. H. Saito, Research Institute for Applied Microorganisms, Tokyo University) was cultivated on an L-agar plate overnight. The cultivated microorganism was inoculated in medium I, that is Spizizen minimal medium (a mixture containing 1.4% of $K_2HPO_4$, 0.6% of $KH_2PO_4$, 0.2% of $(NH_4)_2SO_4$, 0.1% of sodium citrate, 0.02% of $MgSO_4 \cdot 7H_2O$, 0.5% of glucose, 0.02% of casein hydrolysate, 0.2% of yeast extract and 50 $\mu$g/ml of L-tryptophane), and was cultivated on a shaker at 37°C for approximately 4 hours until the cultivation reached the stationary phase. Medium II (a medium similar to medium I except that the concentration of L-tryptophane and yeast extract are 5 $\mu$g/ml and 0.02% respectively and 5 mM of $MgSO_4$ is added) was employed to decrease the concentration of the cultivated cells to 1/10, before the cultivation was continued. A 90-minute period was needed to allow the cells to be converted into competent ones. 0.9 ml of these competent cells were admixed with 0.1 ml of the DNA solution of the chimeric plasmid pHY460 produced above in this EMBODIMENT and were cultivated on a shaker at 37°C for 90 minutes.

      The abovementioned cultivation process allowed the competent cells to be transformed with the DNA to express the genetic information thereof.

(ii)   Detection of transformant

The transformants retaining the plasmid DNA
(pHY460) were applied on an L-agar plate containing
tetracycline at 20 $\mu$g/ml, and were cultivated at 37°C
for 24 hours.  A process similar to that which has been
described in the   description of EMBODIMENT 1 was
applied to some of the colonies acquired above,
in order    to determine the dimension of a plasmid DNA
retained in each transformant.  As a result, the
molecular weight of the chimeric plasmid produced by
the aforementioned process was determined to be approximately 4.6 Md.

E. coli C600r$^-$m$^-$ was also determined to have been
transformed, by means of a process similar
to that    described in (5) for EMBODIMENT 1.

The foregoing description confirms     that the
chimeric plasmid pHY460   can        function as a
shuttle vector for a host-vector system employing at
least E. coli and B. subtilis.

The transformed E. coli (pHY460) and the transformed B. subtilis (pHY460) produced in this EMBODIMENT,
were deposited at Fermentation Research Institute
Agency of Industrial Science and Technology, and the
former was given the deposition No. FERM BP-438 and
the latter was given the deposition No. FERM BP-439.

EMBODIMENT 4     CHIMERIC PLASMID, pHY360

A process similar to that which has been described in (3) for EMBODIMENT 1 was employed to allow the restriction enzymes SacII and BstEII to produce DNA fragments having a pair of cohesive ends by cleaving pHY460, the product of EMBODIMENT 3, and the produced DNA fragments were digested with S1 nuclease to convert both the cohesive ends of each of the fragments into flush ends by nipping off a single stranded DNA chain protruding at each end of the same fragment, before the both flush ends were ligated by T4 ligase to produce a circular DNA.

The abovementioned S1 nuclease digestion was carried out by S1 nuclease having an activity of 50 units/$\mu$l at 37°C for 5 minutes in an S1 reaction buffer containing 30 mM of sodium acetate (pH 4.6), 50 mM of NaCl, 1 mM of ZnSO$_4$ and 5% of glycerol.

A process similar to that which has been described in (4) for EMBODIMENT 1 was employed to transform E. coli C600r$^-$m$^-$ with the plasmid DNA (pHY360) produced above, in order to produce a transformant having the resistance against tetracycline and ampicillin.

A process similar to that which has been described in (5) for EMBODIMENT 1 was employed to determine the dimension of the plasmid DNA retained in the transformant produced above. The results showed that all the transformants had a molecular weight of approximately 3.6 Md.

A process similar to that which has been described in (2) for EMBODIMENT 3 was employed to produce a variety of transformants of B. subtilis.

In this manner, a new chimeric plasmid whose cleavage map is shown by (F) in Fig. l(B) was synthesized from pHY460 whose cleavage map is shown by (E) in Fig. l(B). This new chimeric plasmid was named pHY360.

The aforementioned aquisition of the transformant has proved that this pHY360 can function as a cloning vector (a shuttle vector) at least both for E. coli and for B. subtilis.

The transformed E. coli (pHY360) and the transformed B. subtilis (pHY360) were deposited at Fermentation Research Institute Agency of Industrial Science and Technology, and the former was given the deposition No. FERM BP-443 and the latter was given the deposition No. FERM BP-441.

### EMBODIMENT 5   CHIMERIC PLASMID, pHY310

A process similar to that which has been described in (3) for EMBODIMENT 1 was employed to allow the restriction enzymes AccI and BamHI to cleave pHY360, the product of EMBODIMENT 4.

The reaction mixture containing the cleaved DNA fragments was admixed with T4 DNA polymerase and deoxyribonucleoside triphosphate to convert both the cohesive ends of each of the DNA fragments into flush ends by patching up the vacant sequence complementary to that of a single stranded DNA chain protruding at each end of the same fragment. Namely, the abovementioned reaction was carried out at 37°C for 15 minutes in the mixture of T4 DNA polymerase having the activity of 1 unit, 25 $\mu$M each of

4 kinds of deoxyribonucleoside triphosphate and 20 /l of a buffer having the pH value 8.0 and containing 67 mM of Tris·HCl, 6.7 mM of $MgCl_2$ and 6.7 mM of $\beta$-mercaptoethanol. A subsequent ligation process in which T4 ligase and HindIII linker (dpCAAGCTTG) (produced by Takara Shuzo Co., Ltd) having an optical density of 0.01 OD were added, allowed the HindIII linker to bridge both the flush ends of the linear DNA fragment produced in the above process in order to produce a circular DNA.

A process similar to that which has been described in (4) for EMBODIMENT 1 was employed to transform E. coli with the aforementioned ligated circular DNA. Produced by the process was a transformant having the resistance against tetracycline and ampicillin.

Processes similar to those which have been described for (5) for EMBODIMENT 1 were employed to determine the molecular weight and the recognition and cleavage site for the bridging HindIII linker. The results showed that the approximate molecular weight of the plasmid DNA extracted from the transformant was 3.1 Md and that the same DNA can be cleaved by HindIII.

In this manner, a new chimeric plasmid whose cleavage map is shown by (F') in Fig. 4 was synthesized from pHY360 whose cleavage map is shown by (F) in Fig. 4. This new chimeric plasmid was named pHY310.

As in the cases of pHY460 and pHY360, this pHY310 was determined to have the function to frequently transform also B. subtilis.

The transformed E. coli (pHY310) and the transformed B. subtilis (pHY310) were deposited at Fermentation Research Institute Agency of Industrial Science and Technology.  The former was given the deposition No. FERM BP-436 and the latter was given the deposition No. FERM BP-437.

EMBODIMENT 6    CHIMERIC PLASMID, pHY385

A process similar to that which has been described in (2) for EMBODIMENT 3 was employed to transform B. subtilis with pHY460.  The transformants picked up from some of colonies were cultivated separately in L-broth at 37°C for 50 successive generations.  As in the case of (2) for EMBODIMENT 3, the later cultivations were carried out on an L-agar plate medium containing 20 $\mu$g/ml of tetracycline.

A process similar to that which has been described in (5) for EMBODIMENT 1 proved that the colony which had been obtained by the above cultivation contained a plasmid DNA.

As a result, the plasmid DNA-s having a  molecular weight of 3.85 Md were found in 4 colonies of transformants out of 36 colonies thereof.  Further efforts were used to analyze the structure of this plasmid DNA.  The results showed that this plasmid DNA was a new chimeric plasmid which had been produced from pHY460 by depriving the same plamid DNA of the DNA fragment, the approximate molecular weight of which was 0.75 Md.

In this manner, a new chimeric plasmid whose cleavage map is shown by (G) in Fig. l(B) was synthesized from
pHY460 whose cleavage map is shown by (E) in Fig. l(B).
This new chimeric plasmid was named pHY385.

Processes similar to those which have been described
in (4) and (5) for EMBODIMENT 1 proved that E. coli C600r⁻m⁻
had been transformed with pHY385. This means that this
pHY385 . can function as a cloning vector in the
host-vector system at least both for E. coli and for
B. subtilis.

The transformed E. coli (pHY385) and the transformed
B. subtilis (pHY385) were deposited at Fermentation Research
Institute Agency of Industrial Science and Technology. The
former was given the deposition No. FERM BP-444 and the
latter was given the deposition No. FERM BP-442.

### EMBODIMENT 7    CHIMERIC PLASMID, pHY340

A process similar to that which has been described in
(3) for EMBODIMENT 1 was employed to allow the restriction
enzyme HaeII to cleave pHY385, the product of EMBODIMENT 6,
and to allow T4 ligase to ligate the cleaved DNA fragment.

A process similar to that which has been described in
(4) for EMBODIMENT 1 was employed to transform E. coli C600r⁻m⁻
with the plasmid DNA (pHY340)produced above, in order
to produce a transformant having the resistance against
tetracycline and ampicillin.

A process similar to that which has been described in
(5) for EMBODIMENT 1 was employed to determine the dimension
of the plasmid DNA-s retained in the transformants produced above. The results showed that all the transformants had the plasmid DNA, the approximate molecular weight of which was 3.4 Md.

A process similar to that which has been described in (2) for EMBODIMENT 3 was employed to produce a variety of transformants of B. subtilis.

In this manner, a new chimeric plasmid whose cleavage map is shown by (G') in Fig. 2 was synthesized from pHY385 whose cleavage map is shown by (G) in Fig. 2. This new chimeric plasmid was named pHY340. -

The aforementioned reciprocal transformation processes have proved that this pHY340 can function as a cloning vector (a shuttle vector) at least both for E. coli and for B. subtilis.

The transformed E. coli (pHY340) and the transformed B. subtilis (pHY340) were deposited at Fermentation Research Institute Agency of Industrial Science and Technology. The former was given the deposition No. FERM BP-445 and the latter was given the deposition No. FERM BP-446.

### EMBODIMENT 8    CHIMERIC PLASMID, pHY330

pHY460, the product of EMBODIMENT 3, was cleaved by the restriction enzyme XbaI. After being treated with S1 nuclease, the cleaved DNA fragments were ligated by T4 ligase to synthesize a chimeric plasmid. Namely, $0.5 \mu g/50 \mu l$ of the plasmid DNA (pHY460) was stored at 4°C in buffer A for 4 months, in order to allow each of the plasmid DNA-s to incur "nicks" at random locations thereof and for the ultimate purpose of selectively producing a circular DNA having a desired dimension by ligating a variety of DNA fragments which were shortened in random length.

The plasmid DNA-s having "nicks" were cleaved by the restriction enzyme XbaI having an activity of 4 units dissolved in a buffer containing 10 mM of Tris - HCl (pH 7.6), 7 mM of $MgCl_2$, 7 mM of $\beta$-mercaptoethanol and 50 mM of NaCl. The buffer containing the cleaved DNA fragments was admixed with NaCl in the volume required to adjust the NaCl concentration to 100 mM and with cold ethanol in quantity twice as much as the foregoing concentration adjusted buffer, to be cooled at -20°C. The DNA fragments were collected by 3-minute treatment at 4°C at 15,000 rpm centrifugation, before being washed by cold ethanol. After ethanol was evaporated, the DNA fragments were dissolved in 50 $\mu$1 of S1 buffer containing 30 mM of sodium acetate (pH 4.6), 50 mM of NaCl, 1 mM of $ZnSO_4$ and 5% of glycerol in order to allow S1 nuclease to cleave each of the DNA fragments at "nicks" where it was selectively subjected to the digestion by S1 nuclease due to the single stranded structure thereof.

The DNA fragments were subjected to reaction with 0.5 $\mu$1 of S1 nuclease having an activity of 500 units/$\mu$1 at 37°C for 5 minutes. This reaction mixture was admixed with 100 $\mu$1 of phenol saturated with buffer A. After being agitated, the mixture was centrifuged at room temperature at 15,000 rpm for 3 minutes, to collect the upper layer containing the DNA fragments.

The NaCl concentration of the solution taken out of the upper layer was adjusted to 100 mM. This solution was cooled at -20°C with 200 $\mu$l of -20°C ethanol for 30 minutes, before being centrifuged at 4°C at 15,000 rpm for 3 minutes to collect the precipitate. After the precipitate was washed with cold ethanol, the ethanol was evaporated.

The DNA precipitated at the bottom of the centrifuge tube was dissolved in 20 $\mu$l of water, before being admixed with 3 $\mu$l of 10 mM ATP, 3 $\mu$l of 100 mM dithiothreitol and 3 $\mu$l of 660 mM Tris - HCl (pH 7.6) -6.6 mM MgCl$_2$. T4 ligase having an activity of 10 units was allowed to ligate the DNA fragments at 15°C.

The ligated DNA was admixed with water of 170 $\mu$l which is enough to make the volume 200 $\mu$l. A process similar to that which has been described in (4) for EMBODIMENT 1 was employed to transform 100 $\mu$l of competent cells which had been produced by treating E. coli C600r$^-$m$^-$ with CaCl$_2$, with 100 $\mu$l of the DNA solution. A process similar to that which has been described in (5) for EMBODIMENT 1 was employed to extract the retained plasmid DNA whose molecular weight is approximately 3.3 Md from the transformants.

In this manner, a new chimeric plasmid whose cleavage map is shown by (H) in Fig. 3 was synthesized from pHY460 whose cleavage map is shown by (E) in Fig. 3. This new chimeric plasmid was named pHY330.

Further, pHY330 has been proved to be a cloning vector useful at least for E. coli.

EMBODIMENT 9    CHIMERIC PLASMID, pHY285

A process similar to that which has been described in (3) to        (5) for EMBODIMENT 1 was employed to cleave pHY330, the product of EMBODIMENT 8, employing   a   mixture containing the restriction enzyme HaeII in a buffer containing 10 mM of Tris - HCl (pH 7.6), 7 mM of $MgCl_2$ and 7 mM of $\beta$-mercapto-ethanol, ligate the cleaved DNA fragments produced above and transform E. coli with the ligated DNA produced above, in   order     to produce a circular DNA whose approximate molecular weight is 2.85 MD.

In this manner, a new chimeric plasmid whose cleavage map is  shown by (I) in Fig. 3 was synthesized from pHY330 whose cleavage map is. shown by (H) in Fig. 3.  This new chimeric plasmid was named pHY285.

Further, pHY285 was proved to be a cloning vector useful at least for E. coli.

EMBODIMENT 10    CHIMERIC PLASMID, pHY225

The restriction enzymes SacII and EcoRI were allowed to cleave pHY285, the product of EMBODIMENT 9.  As       in the case of EMBODIMENTS4 and 8, the cleaved DNA fragments were digested with S1 nuclease to convert both the cohesive ends of each of the fragments into   . flush ends by nipping off a single stranded DNA chain protruding at each end of the same fragment, before both the flush ends were ligated by T4 ligase to produce a circular DNA.  Namely, 0.4 $\mu$g/50 $\mu$l of the DNA (pHY285) was cleaved by SacII having  an activity of 4 units dissolved in a buffer containing 10 mM of Tris - HC1 (pH 7.6), 7mM of $MgCl_2$ and 7 mM of $\beta$-mercaptoethanol. The cleavage process was carried out at 37°C for 60 minutes, before the mixture was heated at $60^{\circ}C$ for 10 minutes to terminate the reaction. The reaction mixture in which the DNA-s were dissolved was admixed with NaCl in the amount required to bring the concentration thereof to 40 mM, before being admixed with EcoRI having an activity of 4 units, allowing the DNA-s to be subjected to reaction at 37°C for 60 minutes. After being heated at 60°C for 10 minutes, the reaction mixture was admixed with 50 $\mu$ 1 of water.

This mixture was admixed with phenol saturated with buffer A and shaken, before being centrifuged at room temperature at 15,000 rpm for 30 minutes.

The upper layer containing the DNA-s which was separated from the mixture in the abovementioned centrifugation, was admixed with NaCl in the amount required to adjust the NaCl concentration to 100 mM, before being treated with cold ethanol. After the ethanol was evaporated, the DNA-s were treated with S1 nuclease at 37°C for 5 minutes. A process similar to that which has been described in (3) to (5) for EMBODIMENT 1 was employed to ligate the above produced DNA fragment and to transform E. coli with the DNA, to produce a new chimeric plasmid whose approximate molecular weight is 2.25 Md. In the aforementioned processes, a new chimeric plasmid was synthesized from pHY285 whose cleavage map is shown by (J) in Fig. 3. This new chimeric plasmid was named pHY225. Further, pHY225 was proved to be a cloning vector

at least for <u>E.</u> <u>coli</u>.

Although the present invention has been described with reference to specific chimeric plasmids, pHY780, pHY600, pHY460, pHY385, pHY360, pHY340, pHY330, pHY310, pHY285 and pHY225, this description is not meant to be construed in a limiting sense. Various modifications of the described embodiments, as well as other embodiments of this invention, will become apparent to persons skilled in the art upon reference to the description of the present invention. It is therefore contemplated that the apended claims will cover any such modifications or embodiments as fall within the true scope of this invention.

CLAIMS

1.    A chimeric plasmid comprising:
      (a) a tetracycline resistant gene deriving from
      pAMα1, one of the plasmids retained by
      <u>Streptococcus</u> <u>faecalis</u>,
      (b) an ampicillin  resistant gene deriving from
      pACYC177, one of the vectors applicable to
      <u>Escherichia</u> <u>coli</u>, and
      (c) at least one of (i) an area at which
      replication originates deriving from said plasmid
      pAMα1, and (ii) an area at which replication
      originates deriving from said vector pACYC177,
said tetracycline resistant gene containing only one site
at which said chimeric plasmid is recognized and
cleaved by the restriction enzyme <u>Bal</u>I and said
ampicillin resistant gene containing only one site at
which said chimeric plasmid is recognized and cleaved by
the restriction enzymes <u>Bgl</u>I and <u>Pst</u>I.

2.    A chimeric plasmid according to claim 1, denoted
pHY780 herein, whose approximate molecular weight is
7.8 Md and whose cleavage map is shown in Fig. 5 of the
Drawings.

3.    A chimeric plasmid according to claim 1, denoted
pHY600 herein, whose approximate molecular weight is
6.0 Md and whose cleavage map is shown in Fig. 6 of the
Drawings.

4.    A chimeric plasmid accoridng to claim 1, denoted
pHY460 herein, whose approximate molecular weight is
4.6 Md and whose cleavage map is shown in Fig. 7 of the
Drawings.

5.    A chimeric plasmid according to claim 1, denoted
pHY385 herein, whose approximate molecular weight is
3.85 Md and whose cleavage map is shown in Fig. 8 of
the Drawings.

6.    A chimeric plasmid according to claim 1, denoted

0115936

- 53 -

pHY360 herein, whose approximate molecular weight is
3.6 Md and whose cleavage map is shown in Fig. 9 of the
Drawings.

7.    A chimeric plasmid according to claim 1, denoted
pHY340 herein, whose approximate molecular weight is
3.4 Md and whose cleavage map is shown in Fig. 10 of the
Drawings.

8.    A chimeric plasmid according to claim 1, denoted
pHY330 herein, whose approximate molecular weight is
3.3 Md and whose cleavage map is shown in Fig. 11 of
the Drawings.

9.    A chimeric plasmid according to claim 1, denoted
pHY310 herein, whose approximate molecular weight is
3.1 Md and whose cleavage map is shown in Fig. 12 of
the Drawings.

10.    A chimeric plasmid according to claim 1, denoted
pHY285 herein, whose approximate molecular weight is
2.85 Md and whose cleavage map is shown in Fig. 13 of
the Drawings.

11.    A chimeric plasmid according to claim 1, denoted
pHY225 herein, whose approximate molecular weight is
2.25 Md and whose cleavage map is shown in Fig. 14 of
the Drawings.

12.    A process for preparing a chimeric plasmid claimed
in any one of the preceding claims, which process
comprises cleaving pAMα1, a plasmid retained by
Streptococcus faecalis DS5 (ATCC14508), and pACYC177,
a vector applicable to Escherichia coli, and ligating
the cleaved DNA fragments of said plasmid and vector to
include a tetracycline resistant gene (Tc) deriving
from the aforementioned pAMα1, an ampicillin resistant
gene (Amp) deriving from the aforementioned pACYC177
and either or both of: (i) an area at which replication
originates deriving from said plasmid pAMα1, and (ii)
an area at which replication originates deriving from
said vector pACYC177.

13.    A method employing a vector to convey a gene into a host cell, to retain and express the gene therein, in which method there is used as vector a chimeric plasmid claimed in any one of claims 1-11 or prepared by a process claimed in claim 12.

14. A microorganism as deposited at the Fermentation Research Institute Agency of Industrial Science & Technology, Japan, under the deposition number FERM BP-436, FERM BP-437, FERM BP-438, FERM BP-439, FERM BP-441, FERM BP-442, FERM BP-443, FERM BP-444, FERM BP-445 or FERM BP-446.

15. Biosynthesis employing a gene cloned by a host into which the gene has been conveyed by a method claimed in claim 13.

# FIG. 1 (A)

0115936

2/15

FIG. I(B)

# FIG. 2

(G)

(G')

FIG. 3

0115936

4/115

0115936

5/15

F I G . 4

(F)

pHY360
3.6Md

Hinc II
Ori177
SacII/BstE II
BamHI
Amp
EcoRI
Acc I
Tc
OripAMα1

Acc I
BamHI

Hind III Linker

(F')

pHY310
3.1Md

Hinc II
Ori 177
Hind III
Acc I/BamHI
Amp
Tc
OripAMα1

FIG. 5

0115936

6/15

0115936

7/15

F I G. 6

0115936

8/15
F I G. 7

FIG. 7

pHY 460
4.6 Md

0115936

9/15

F I G. 8

10/15
FIG. 9

- Hinc II
- Pvu I
- Pst I
- Bgl I
- Ban I

BamH I
Ava I
Xba I

3

0

Amp

Ori 177

pHY 360

3.6 Md

I

Hinc II

EcoR I
Acc I

OripAMα1

Tc

EcoR V

2

Bal I

HincII · Hpa I

# F I G. 10

0115936

12/15

FIG. 11

pHY 330
3.3Md

Hinc II
Pvu I
Pst I
Bgl I
Ban I
Amp
Ori 177
Sac II
Acc I
Hae II
Hae II
EcoR I
BstE II
Hinc II
EcoR V
Bal I
HpaI · HincII
Tc
3
0
1
2

13/15

F I G. 12

Hinc II
Pvu I
Pst I
Bgl I
Ban I
Hind III
Ori177
OripAMαI
pHY 310
3.1 Md
Amp
3  0
Hinc II
1
2
Tc
EcoR V
Bal I
Hpa I · Hinc II

0115936

14/15

F I G. 13

0115936

15/15
FIG. 14

pHY 225
2.25 Md

Ori 177

Hinc II
Pvu I
Pst I
Bgl I
Ban I
Amp
BstE II
Hinc II
EcoR V
Bal I
Hpa I · Hinc II
Tc

**European Patent Office**

**EUROPEAN SEARCH REPORT**

Application number

| | DOCUMENTS CONSIDERED TO BE RELEVANT | | EP 84300403.7 |

| Category | Citation of document with indication, where appropriate, of relevant passages | Relevant to claim | CLASSIFICATION OF THE APPLICATION (Int. Cl. ³) |
|---|---|---|---|
| A,D | PROCEEDINGS OF THE NATIONAL ACADEMY OF SCIENCES OF THE UNITED STATES OF AMERICA, vol. 72, no. 5, May 1975, Baltimore, USA<br><br>D.B. CLEWELL et al.: "Plasmid--Determined Tetracycline Resistance in Streptococcus faecalis: Evidence for Gene Amplification During Growth in Presence of Tetracycline" pages 1720-1724<br><br>* Abstract * | 1,12 | C 12 N 15/00//<br>C 12 R 1/46<br>C 12 R 1/19 |
| A,D | JOURNAL OF BACTERIOLOGY, vol. 134, no. 3, June 1978, Washington, DC, USA<br><br>A.C.Y. CHANG et al.: "Construction and Characterization of Amplifiable Multicopy DNA Cloning Vehicles Derived from the P15A Cryptic Miniplasmid" pages 1141 - 1156<br><br>* Pages 1141, 1143 * | 1,12 | |
| A,P | EP - A2 - 0 085 548 (ELI LILLY AND COMPANY)<br><br>* Claims 1,18 * | 1,12 | |

TECHNICAL FIELDS SEARCHED (Int. Cl. ³)

C 12 N

The present search report has been drawn up for all claims

| Place of search | Date of completion of the search | Examiner |
|---|---|---|
| VIENNA | 26-04-1984 | WOLF |